# EUROPÄISCHE PATENTANMELDUNG

(11) **EP 1 197 559 A2**
(43) Veröffentlichungstag der Anmeldung: **17.04.2002**
(21) Anmeldenummer: 01123537.1
(22) Anmeldetag: 29.09.2001
(51) Int. Cl.: C12P 7/62, C12P 7/42, C12P 7/64, C07C 69/00, A61K 7/00

(54) **Enzymatische Herstellung von bei Raumtemperatur festen Hydroxyfettsäureestern mehrwertiger Alkohole**

(30) Priorität: 12.10.2000 DE 10050403
(71) Anmelder: Goldschmidt AG, 45127 Essen (DE)
(72) Erfinder: Grüning, Burghard, Dr., 45134 Essen (DE); Hills, Geoffrey, 45355 Essen (DE); Lersch, Peter, Dr., 46537 Dinslaken (DE)

(57) **Zusammenfassung**

Gegenstand der Erfindung ist ein Verfahren zur Herstellung von bei Raumtemperatur festen Hydroxyfettsäureestern mit verbessertem Schmelzverhalten, hergestellt gemäß einem Verfahren, bei welchem in Gegenwart eines Enzyms mindestens zwei primäre und gegebenenfalls sekundäre oder tertiäre Hydroxylgruppen aufweisende Di- oder Polyole und Hydroxyfettsäuren und/oder Hydroxyfettsäurealkylester, gegebenenfalls in einem Lösungsmittel, gegebenenfalls bei gegenüber der Atmosphäre vermindertem Druck, und Temperaturen zwischen 20 °C und 110 °C und unter Entfernung des entstehenden Reaktionswassers oder des Reaktionsalkohols, umgesetzt werden.

## Beschreibung

Gegenstand der Erfindung ist ein Verfahren zur enzymatischen Herstellung von bei Raumtemperatur festen Hydroxyfettsäureestern mit verbessertem Schmelzverhalten durch Umsetzung von Hydroxyfettsäuren mit mehrwertigen, mindestens zwei primäre und gegebenenfalls weitere sekundäre oder tertiäre Hydroxylgruppen aufweisenden Alkoholen.

Hydroxyfettsäureester von Polyolen finden vielfach Verwendung in solchen Anwendungen, in denen ihre grenzflächenaktiven Eigenschaften zum Tragen kommen. Als Beispiele seien ihre Verwendung als Emulgatoren oder Dispergiermittel in Lacken und Farben oder in kosmetischen Präparaten genannt.

Besonders häufig verwendete Produkte stellen die Ricinol- und Hydroxystearinsäureester des Glycerins und des Polyglycerins dar (DE 44 20 516). Die Glycerinester der Ricinolsäure oder deren hydrierte Derivate, die Glycerinester der 12-Hydroxystearinsäure, sind in der Regel natürlichen Ursprungs.

Weiterhin ist der Einsatz von Estern der Hydroxystearinsäure als Geliermittel beziehungsweise Gelbildner in Deodorant-Stiften beziehungsweise Antiperspirant-Gelen in den WO 98/58623, WO 98/27952, US 5,744,130 erwähnt.

Die Herstellung von Hydroxyfettsäureestern auf chemischem Wege durch Veresterung bzw. Umesterung von Hydroxyfettsäuren oder deren Ester mit Alkoholen gelingt nur bei höheren Temperaturen in Gegenwart von Katalysatoren wie beispielsweise organischen und anorganischen Zinnverbindungen, Titanverbindungen, Blei-, Zinn-, Zinkseifen, Schwefelsäuren, Aryl-, Alkylsulfonsäuren und Reaktionszeiten bis zu 20 Stunden (vergl. J. Am. Pharm. Assoc. 32, (1943), S. 115 - 118; JP-A-79-56063 ,CA 94:120899; DD 155771; JP-A-78-14970, CA 101:151437).

Bei der Synthese von Ethylenglykol-, Propylenglykol- sowie Trimethylenglykolestern der Hydroxystearinsäure mit p-Toluolsulfonsäure in Toluol unter Rückfluß bei 135 bis 145 °C konnten relativ reine Produkte nur durch extensive Fraktionierung, beispielsweise in Ether, isoliert werden. Ausbeuten von nur 40 bis 60 % wurden erhalten, mit erheblichen Mengen an niedrig schmelzendem, amorphem Material (J. Am. Pharm. Assoc. 32, (1943), S. 115 - 118).

Abgesehen davon, dass diese Katalysatoren - insbesondere die schwermetallhaltigen - nur in begrenztem Umfang heute noch anwendbar sind, weisen sie eine Reihe weiterer Nachteile auf, wie ungenügende Löslichkeit in den Edukten und/oder Reaktionsprodukten, geringe katalytische Aktivität bei gleichzeitig geringer Selektivität. Insbesondere sind unter diesen Bedingungen unerwünschte Nebenreaktionen wie Dehydratisierungsreaktionen oder Veresterungen der Hydroxylgruppen in den Hydroxyfettsäuren zu Polyhydroxyfettsäuren nicht oder nicht ausreichend zu verhindern.

In jüngerer Zeit sind in der Literatur vermehrt Ver- bzw. Umesterungsreaktionen von Hydroxyfettsäuren mit Alkoholen unter Mitverwendung von Enzymkatalysatoren beschrieben worden. Als Vorteile wurden die milderen Reaktionsbedingungen und die Stereo- bzw. Regiospezifizität sowie der damit verbundene höhere Reinheitsgrad der Reaktionsprodukte genannt.

In JAOCS, Vol.73, No.11 (1996), Seite 1513 ff. werden zur Erzielung hoher Ausbeuten die Vinylester der Hydroxystearinsäure eingesetzt. Die Verwendung von Vinylestern ergibt aufgrund der irreversiblen Umesterung zwar hohe Umsätze, sie führt jedoch zugleich zur Bildung des Nebenproduktes Acetaldehyd, der praktisch nicht vollständig zu entfernen ist. Acetaldehyd ist aus toxikologischen Gründen und wegen seines Geruchs insbesondere in kosmetischen Produkten unerwünscht.

In J. Agric. Food Chem. 1988, 46, 2427 ff. werden Untersuchungen über die Reaktivität und Substratselektivität bei der Veresterung von verschiedenen ungesättigten Fettsäuren und beispielsweise 12-Hydroxystearinsäure und Ricinolsäure mit 1-Butanol durchgeführt. Der Umsetzungsgrad für 12-Hydroxystearinsäure liegt nach ca. 160 Stunden unterhalb 30 %.

Gemäß JAOCS, Vol. 75 No. 8 (1998), 1075 ff. wurde gefunden, dass die Lipase- katalysierte Umsetzung von 12-Hydroxystearinsäure mit langkettigen C₈- bis C₁₈-Fettalkoholen durchgeführt werden kann und dass die Schmelzpunkte der reinen Reaktionsprodukte deutlich niedriger liegen als die der Ausgangsmischungen.

Demgegenüber bestand die Aufgabe der vorliegenden Erfindung darin, ein verbessertes enzymatisches Verfahren zur Herstellung von bei Raumtemperatur festen Hydroxyfettsäureestern zur Verfügung zu stellen, welches Produkte mit verbessertem Schmelzverhalten und mit erhöhten Ausbeuten - insbesondere im Hinblick auf technisch relevante Maßstäbe - liefert. Die Herstellung solcher Ester konnte im technischen Maßstab bislang nicht befriedigend gelöst werden. Relativ reine Produkte können nur mit großem Aufwand über zum Teil mehrstufige Reinigungsverfahren gewonnen werden (beispielsweise J. Am. Pharm. Assoc. 32, (1943), S. 115 - 118).

Die vorgenannte Aufgabe wird gelöst durch ein Verfahren zur enzymatischen Herstellung von bei Raumtemperatur festen Hydroxyfettsäureestern mit verbessertem Schmelzverhalten, wobei man eine Reaktionsmischung aus Hydroxyfettsäuren und/oder deren Alkylester mit C₁- bis C₆-, vorzugsweise C₁- bis C₃-Monoalkoholen und einem oder mehreren Polyolen, gegebenenfalls in einem geeigneten Lösungsmittel, in Gegenwart eines Enzyms, welches die Veresterungs- bzw. Umesterungsreaktion katalysiert, nach Erreichen der erforderlichen bzw. gewünschten Reaktionstemperatur im Bereich von ca. 20 bis ca. 110 °C, bevorzugt 40 bis 90 °C, gegebenenfalls bei gegenüber der Atmosphäre vermindertem Druck, vorzugsweise kleiner 400 mbar, insbesondere kleiner 100 mbar, unter gegebenenfalls kontinuierlicher Entfernung des entstehenden Reaktionswassers und/oder des Reaktionsalkohols umsetzt.

Das erfindungsgemäße Verfahren ermöglicht die Herstellung von Hydroxyfettsäureestern in einem technisch akzeptablen Maßstab mit gegenüber dem Stand der Technik erhöhten Reinheiten oft größer 80 %, teilweise größer 90 %. Die Reaktionsmischungen enthalten keine bedenklichen Nebenprodukte wie Acetaldehyd und nur wenig Kondensationsprodukte der Hydroxystearinsäure mit sich selbst. Die erfindungsgemäß hergestellten Ester weisen allgemein ein verbessertes Schmelzverhalten auf. Die Eigenschaft verbessertes Schmelzverhalten ist im Vergleich zum Stand der Technik zu verstehen und bedeutet einen höheren Schmelzpunkt, dargestellt beispielsweise durch den Tropfpunkt, und die Abwesenheit niedrig schmelzender Anteile, die eine Konfektionierung wie zum Beispiel Pelletierung erschweren.

Ein wesentlicher Vorteil der erfindungsgemäß hergestellten Ester gegenüber den weichen und zum Teil noch klebrigen oder fließfähigen Mischungen des Standes der Technik liegt darin, dass sie bei Raumtemperatur fest sind und sich daher leicht in fester Form etwa als Pulver, Schuppen oder Pellets handhaben lassen.

Die Umsetzung kann beispielsweise in einem Rührkesselreaktor oder in einem Festbettreaktor durchgeführt werden.

Der Rührkesselreaktor ist vorzugsweise mit einer Vorrichtung zum Abdestillieren des freigesetzten Wassers oder Alkohols ausgestattet. Die Reaktion wird durchgeführt, bis der gewünschte Umsatz erreicht ist. Nach beendeter Umsetzung kann der Enzymkatalysator durch geeignete Maßnahmen wie Filtrieren oder Dekantieren abgetrennt werden und gegebenenfalls mehrmals wiederverwendet werden.

Der Festbettreaktor kann beispielsweise mit immobilisierten Enzymen bestückt sein, wobei die Reaktionsmischung durch den mit dem Katalysator gefüllten Reaktor gepumpt wird. Bei kontinuierlicher Arbeitsweise wird die Reaktionsmischung nur einmal durch den Festbettreaktor gefördert, wobei mit der Fließgeschwindigkeit die Verweilzeit und damit der gewünschte Umsatz zu steuern ist. Es ist auch möglich die Reaktionsmischung solange im Kreislauf durch den Festbettreaktor zu pumpen, bis der gewünschte Umsatz erreicht ist.

Mit einem auf einem Träger immobilisierten Enzym kann die Reaktion auch in einem Wirbelbettreaktor durchgeführt werden.

Das gebildete Wasser oder der gebildete Alkohol kann gegebenenfalls unter Anwendung von Unterdruck vorzugsweise in einem nachgeschalteten Reaktor entfernt werden. Das Kondensat kann aber auch durch andere übliche Methoden wie Pervaporation oder durch Einsatz von Trockenmitteln oder Molekularsieben aus dem Reaktionsgleichgewicht entfernt werden.

Die Reaktion wird vorzugsweise in Lösungsmitteln durchgeführt. Die Art des Lösungsmittels kann im Sinne der vorliegenden Erfindung in weiten Grenzen variiert werden. Besonders bevorzugt im Sinne der vorliegenden Erfindung werden Lösungsmittel oder deren Mischungen eingesetzt wie beispielsweise Cyclohexan, Methylcyclohexan, Decalin, 2-Methyl-2-Butanol oder Benzinfraktionen oder N-Methylpyrrolidon. Gegebenenfalls kann auch ein flüssiger Reaktand als Lösungsmittel dienen.

Die Reaktion kann aber auch ohne Lösungsmittel bei Temperaturen in der Schmelze mit Enzymen durchgeführt werden. Hierbei kann das Reaktionswasser oder der gebildete Alkohol einfach unter Vakuum, vorzugsweise < 100 mbar, entfernt werden, um vollständige Umsetzungen zu erreichen.

Im Anschluß an die Reaktion, kann der erhaltene Ester nach an sich bekannten Verfahren aufbereitet werden. Im allgemeinen sind Umsetzungsgrad und Reinheit der technischen Mischungen ausreichend um diese ohne weitere Aufbereitungsstufen in den vorgesehenen Anwendungsgebieten einzusetzen.

Als erfindungsgemäß verwendbare Hydroxyfettsäuren bzw. Hydroxyfettsäureester sind die natürlichen oder synthetischen Fettsäuren und deren Ester anzusehen, welche mindestens eine sekundäre Hydroxylgruppe im Molekül enthalten. Sie entsprechen der allgemeinen Formel R^{a}-COOR^{b}, wobei R^{a} für einen linearen gesättigten Fettsäurerest mit 6 bis 30 C-Atomen steht, der mindestens eine sekundäre aber keine primäre Hydroxylgruppe trägt, R^{b} = H oder ein gesättigter Kohlenwasserstoffrest mit 1 bis 6 C-Atomen, vorzugsweise H oder ein Alkylrest mit 1 bis 3 C-Atomen ist, wie z. B. 12-Hydroxystearinsäure, 8,9-Dihydroxystearinsäure und α-Hydroxyoctansäure und deren Ester.

Als Polyole im Sinne der vorliegenden Erfindung können mehrwertige, mindestens zwei primäre und gegebenenfalls sekundäre oder tertiäre Hydroxylgruppen aufweisende Alkohole der allgemeinen Formel HO-R^{c}-OH verwendet werden, worin R^{c} für einen linearen, gegebenenfalls verzweigten Kohlenwasserstoffrest mit 2 bis 5 C-Atomen steht, oder
- R^{c}: die Bedeutung -CH₂-[CH(OH)]ₖ-CH₂-; [-CH₂-CH₂-O-(CHR'-CH₂-O)ₘ-CH₂-CH₂-]ₙ; -CH₂-CH(OH)-CH₂-(-OCH₂CH(OH)-CH₂)ₚ-; -CH₂-C(C₂H₅)(CH₂OH)-CH₂-; -CH₂-C(CH₂OH)₂-CH₂- hat, mit
- k: 1 bis 4,
- m: 0 bis 20, insbesondere 0 bis 10,
- n: 1 bis 5,
- p: 0 bis 15, vorzugsweise 0 bis 10, insbesondere 1 bis 5 und
- R': H oder ein kurzkettiger Alkylrest, insbesondere -CH₃,
ist.

Bevorzugte Alkohole sind Ethylenglykol, Diethylenglykol, Triethylenglykol, Polyethylenglykol, Glycerin und dessen unter dem Begriff Polyglycerin zusammengefassten Kondensationsprodukte wie insbesondere Diglycerin, Triglycerin und Tetraglycerin; 1,4-Butandiol, Trimethylolpropan, Pentaerythrit und Sorbit.

Das Stoffmengenverhältnis von primären Hydroxylgruppen der Alkohole zu Carboxylgruppen der Hydroxyfettsäuren und/oder deren Ester ist variierbar. In einer besonders bevorzugten Ausführungsform der vorliegenden Erfindung wird bei Alkoholen mit zwei primären OH-Gruppen das Stoffmengenverhältnis von primären Hydroxylgruppen zu Carboxylgruppen und/oder Estergruppen der Fettsäure und/oder Fettsäurealkylester im Bereich von ca. 2,1 Mol zu ca. 1 Mol bis 1 Mol zu ca. 1,1 Mol, bevorzugt 1,1 zu 1 bis 1 zu 1,1 Mol eingestellt. Bei Alkoholen mit 3 oder mehr primären OH-Gruppen richtet sich das Verhältnis nach dem gewünschten Veresterungsgrad, der mindestens 2 sein sollte.

Die gemäß der vorliegenden Erfindung einzusetzenden Enzyme oder Enzymkatalysatoren zur Verwendung in nichtwässrigen Medien sind Hydrolasen, insbesondere Lipasen, welche bevorzugt als Pulver oder auch in immobilisierter Form eingesetzt werden können. Sie sind im Stand der Technik bekannt und im Handel erhältlich.

Besonders bevorzugt im Sinne der vorliegenden Erfindung sind Lipasen, insbesondere ein immobilisiertes Lipasesystem welches unter der Bezeichnung Novozym®435 von der Firma Novo Nordisk im Handel angeboten wird.

Die erfindungsgemäßen Hydroxyfettsäureester der allgemeinen Formel (I) eignen sich für vielfältige Anwendungen zum Beispiel im Lack- und Farbenbereich, in kosmetischen und Reinigungspräparaten, als Geliermittel beziehungsweise Gelbildner in Deodorant-Stiften beziehungsweise Antiperspirant-Gelen sowie in vielfältigen technischen Anwendungen, zur Stabilisierung von Dispersionen, zum Beispiel von Pigmenten, und Emulsionen, zur Verdickung und Gelierung von Lösungsmitteln und Ölen, zur Verbesserung des Hautgefühls.

### Ausführungsbeispiele:

### Beispiel 1:

12-Hydroxystearinsäure (136,5 g) und Ethylenglykol (13,8 g) wurden in Cyclohexan (150 g) bei 60 °C gelöst und 3 g Novozym®435 wurden unter Rühren zugegeben. Mit einem Wasserabscheider wurde das Reaktionswasser unter Rückfluss bei 300 bis 500 mbar Vakuum azeotrop destillativ abgetrennt. Nach 6 h wurde die Reaktion abgebrochen, der Katalysator abfiltriert und Cyclohexan abdestilliert. Die Reinheit des Produktes wird mittels Gelpermeationschromatographie bestimmt: Ethylenglykoldi-12-hydroxystearat 89 %. Das Produkt hat einen Tropfpunkt von 85 °C.

### Beispiel 2:

12-Hydroxystearinsäure (136,5 g) und Ethylenglykol (13,8 g) wurden in 2-Methyl-2-Butanol (250 g) bei 70 °C gelöst und unter Rühren wurden 3 g Novozym®435 zugegeben. Das Reaktionswasser wurde bei 100 bis 200 mbar Vakuum azeotrop destillativ abgetrennt. Nach 9 h wurde die Reaktion abgebrochen, der Katalysator abfiltriert und 2-Methyl-2-Butanol abdestilliert. Die Zusammensetzung des Produktes wird mittels Gelpermeationschromatographie bestimmt. Der Gehalt an Ethylenglykoldi-12-hydroxystearat wurde zu 72 % bestimmt. Das Produkt hatte einen Tropfpunkt von 81 °C.

### Beispiel 3:

12-Hydroxystearinsäure (136,5 g) und Ethylenglykol (13,8 g) wurden ohne Lösungsmittel bei 90 °C gemischt und 3 g Novozym®435 zugegeben. Bei 10 bis 40 mbar wurde das Reaktionswasser entfernt und nach 5 h die Reaktion abgebrochen. Nach Abfiltration des Enzymkatalysators erhielt man ein Produkt mit einem Ethylenglykoldi-(12-hydroxystearat)gehalt von 82 %. Das Produkt hatte einen Tropfpunkt von 85 °C.

### Beispiel 4:

12-Hydroxystearinsäure (122,6 g) und Trimethylolpropan (27,9 g) wurden bei 90 °C gemischt und 3 g Novozym®435 zugegeben. Unter Vakuum von 10 mbar wurde das entstehende Reaktionswasser entfernt und nach 16 h wurde die Reaktion abgebrochen. Das erhaltene Produkt nach Abfiltration des Enzymkatalysators enthielt nach GPC-Analyse 53 % Trimethylolpropandi-12-hydroxystearat. Das Produkt hatte einen Tropfpunkt von 44 °C.

### Beispiel 5:

12-Hydroxystearinsäure (131,2 g) und 1,4-Butandiol (19,2 g) wurden bei 60 °C in 150 g Cyclohexan gelöst und mit 3 g Novozym®435 versetzt. Bei 300 bis 500 mbar wurde das Reaktionswasser mit Hilfe eines Wasserabscheiders azeotrop abdestilliert. Nach 4 h wurde die Reaktion abgebrochen, der Enzymkatalysator abfiltriert und Cyclohexan abdestilliert. Das Produkt enthielt nach GPC-Analyse 88 % Butandioldi-12-hydroxystearat. Es hatte einen Tropfpunkt von 77 °C.

### Beispiel 6:

12-Hydroxystearinsäure (120 g) und Triethylenglykol (30,6 g) wurden bei 90 °C gemischt und mit 3 g Novozym®435 versetzt. Bei ca. 10 mbar wurde das Reaktionswasser entfernt und nach 4 h die Reaktion durch Abfiltration des Enzymkatalysators abgebrochen. Man erhielt ein Produkt mit einem Gehalt von 82 % Triethylenglykoldi-12-hydroxystearat nach GPC-Analyse. Das Produkt hatte einen Tropfpunkt von 65 °C.

### Beispiel 7:

12-Hydroxystearinsäure (117,5 g) und Diglycerin (33,2 g) wurden bei 90 °C gemischt und 3 g Novozym®435 zugegeben. Bei ca. 10 mbar wurde das Reaktionswasser entfernt und nach 6 h die Reaktion durch Abfiltration des Enzymkatalysators abgebrochen. Das erhaltene Produkt enthielt nach GPC-Anaylse 44 % Diglycerindi-12-hydroxystearat. Es hatte einen Tropfpunkt von 75 °C.

### Beispiel 8:

12-Hydroxystearinsäure (136 g) und Ethylenglykol (14 g) wurden in Decalin (150 g) bei 70 °C gelöst und 3 g Novozym®435 unter Rühren zugegeben. Unter 25 bis 40 mbar Vakuum wurde das Reaktionswasser abdestilliert, ohne dass das Decalin gleichzeitig abdestilliert wurde. Nach 6 h wurde der Enzymkatalysator Novozym®435 abfiltriert und das Decalin wurde bei 120 °C unter Vakuum vom Produkt, Ethylenglykoldi-12-hydroxystearat destillativ entfernt. Das Produkt enthielt nach GPC-Analyse 80 % Ethylenglykoldi-12-hydroxystearat. Es hatte einen Tropfpunkt von 82 °C.

### Vergleichsbeispiele 1 bis 6:

Eine Mischung von Alkohol und 12-Hydroxystearinsäure in einem Molverhältnis von 1 : 2 wurde unter Stickstoffdurchleitung auf 180 °C erhitzt. Bei 110 bis 120 °C wurde als Katalysator Zinnoxalat (0,15 Gew.-%) zugegeben. Die Reaktion wurde durchgeführt bis die Säurezahl unter 1 lag (Dauer 4 bis 10 Stunden). Zur Aufarbeitung wurde zu dem Reaktionsprodukt bei 120 °C 0,1 bis 0,3 Gew.-% Filterhilfsmittel Tegotinex®P zugegeben, 1 h gerührt und dann filtriert. Nach dieser allgemeinen Arbeitsweise wurden aus 12-Hydroxystearinsäure die folgenden Ester hergestellt.
- V1: Ethylenglykoldihydroxystearat
- V2: Trimethylolpropantrihydroxystearat
- V3: 1,4-Butandioldihydroxystearat
- V4: Triethylenglykoldihydroxystearat
- V5: Diglycerindihydroxystearat

In der nachfolgenden Tabelle sind die enzymatisch hergestellten Hydroxystearinsäureester den chemisch hergestellten Produkten gegenübergestellt. Jeweils in einer Zeile finden sich daher identische Ausgangsalkohole.

| Beispiel: | Diestergehalt (Gew.-%) | Pour Point (°C) | Tropfpunkt (°C) | Vergleichsbeispiel | Diestergehalt (Gew.-%) | Pour Point (°C) | Tropfpunkt (°C) |
|---|---|---|---|---|---|---|---|
| 1 | 89 | 80 | 85 | V1 | 6 | 0 | n. b.* |
| 4 | 53 | 39 | 44 | V2 | 33 | 32 | 40 |
| 5 | 88 | 73 | 77 | V3 | 39 | 65 | 68 |
| 6 | 82 | 63 | 65 | V4 | 34 | 16 | 49 |
| 7 | 44 | 61 | 75 | V5 | 27 | 51 | 55 |
| n. b. = nicht bestimmbar. Das Produkt war halbflüssig bei 25 °C, klar bei 65 °C. | | | | | | | |

Die Gegenüberstellung zeigt die relativ hohe Reinheit anhand des jeweiligen Di- bzw. Triestergehaltes und das verbesserte Schmelzverhalten der erfindungsgemäß enzymatisch hergestellten Produkte gegenüber den chemisch hergestellten Analoga. Das Schmelzverhalten wurde mittels des "pour point" nach DIN ISO 3016 und des Tropfpunktes nach DIN ISO 2176 bestimmt. Ein niedriger "pour point" ist ein Indiz, dass niedrig schmelzende Anteile vorhanden sind. Ein hoher "pour point" und hoher Tropfpunkt sind vorteilhaft für ein erfindungsgemäß erwünschtes gutes Schmelzverhalten.

## Patentansprüche

1. Verfahren zur Herstellung von bei Raumtemperatur festen Hydroxyfettsäureestern mit verbessertem Schmelzverhalten, bei welchem in Gegenwart eines Enzyms mindestens zwei primäre und gegebenenfalls sekundäre oder tertiäre Hydroxylgruppen aufweisende Alkohole und Hydroxyfettsäuren und/oder Hydroxyfettsäurealkylester gegebenenfalls in einem Lösungsmittel, gegebenenfalls bei gegenüber der Atmosphäre vermindertem Druck, und Temperaturen zwischen 20 °C und 110 °C unter Entfernung des entstehenden Reaktionswassers oder des Reaktionsalkohols umgesetzt werden.

2. Verfahren nach Anspruch 1, **dadurch gekennzeichnet, dass** gesättigte Hydroxyfettsäuren und/oder Hydroxyfettsäurealkylester der allgemeinen Formel R^{a}-COOR^{b} eingesetzt werden, deren Reste R^{a} eine Hydroxylgruppe und 12 bis 24 C-Atome aufweisen und deren Reste R^{b} H oder ein gesättigter Kohlenwasserstoffrest mit 1 bis 6 C-Atomen sind.

3. Verfahren nach einem der Ansprüche 1 oder 2, **dadurch gekennzeichnet, dass** 12-Hydroxystearinsäure und/oder deren Ester mit kurzkettigen monofunktionellen Alkoholen mit 1 bis 3 C-Atomen eingesetzt werden.

4. Verfahren nach einem der Ansprüche 1 bis 3, **dadurch gekennzeichnet, dass** als Polyole Verbindungen der allgemeinen Formel HO- R^{c}-OH eingesetzt werden, worin
R^{c} die Bedeutung -CH₂-[CH(OH)]ₖ-CH₂-; [-CH₂-CH₂-O-(CHR'-CH₂-O)ₘ-CH₂-CH₂-]ₙ; -CH₂-CH(OH)-CH₂-(-OCH₂CH(OH)-CH₂)ₚ-; -CH₂-C(C₂H₅)(CH₂OH)-CH₂-; -CH₂-C(CH₂OH)₂-CH₂- mit
k 1 bis 4,
m 0 bis 20, insbesondere 0 bis 10,
n 1 bis 5,
p 0 bis 15, vorzugsweise 0 bis 10, insbesondere 1 bis 5 und
R' H oder kurzkettiger Alkylrest, insbesondere CH₃,
hat.

5. Verfahren nach Anspruch 4, **dadurch gekennzeichnet, dass** als Polyole Ethylenglykol und die darauf basierende homologe Reihe der Polyethylenglykole oder Glycerin und dessen unter dem Begriff Polyglycerin zusammengefassten Kondensationsprodukte wie Diglycerin, Triglycerin und Tetraglycerin einsetzt werden.

6. Verfahren nach Anspruch 4, **dadurch gekennzeichnet, dass** als Polyole 1,3-Propandiol, 1,4-Butandiol, 1,5-Pentandiol, Trimethylolpropan, Pentaerythrit, oder Sorbit eingesetzt werden.

7. Verfahren nach einem der Ansprüche 1 bis 6, **dadurch gekennzeichnet, dass** man das Stoffmengenverhältnis der primären Hydroxylgruppen zu Carboxylgruppen und/oder Estergruppen im Bereich von 2,1 Mol zu 1 Mol bis 1 Mol zu 1,1 Mol einstellt.

8. Verfahren nach einem der Ansprüche 1 bis 7, **dadurch gekennzeichnet, dass** man Enzyme einsetzt, die ausgewählt sind aus gegebenenfalls immobilisierten Lipasen, Esterasen oder Proteasen.

9. Verfahren nach einem der Ansprüche 1 bis 8, **dadurch gekennzeichnet, dass** man die Reaktion bei einer Temperatur im Bereich von 20 bis 110 °C und unter verminderten Druck durchführt.

10. Verfahren nach einem der Ansprüche 1 bis 9, **dadurch gekennzeichnet, dass** man die Reaktion in Festbettreaktoren durchführt.

11. Technische Mischungen von bei Raumtemperatur festen Hydroxyfettsäureestern mit verbessertem Schmelzverhalten hergestellt gemäß eines Verfahrens bei welchem in Gegenwart eines Enzyms mindestens zwei primäre und gegebenenfalls sekundäre oder tertiäre Hydroxylgruppen aufweisende Di- oder Polyole und Hydroxyfettsäuren und/oder Hydroxyfettsäurealkylester, gegebenenfalls in einem Lösungsmittel, gegebenenfalls bei gegenüber der Atmosphäre vermindertem Druck, und Temperaturen zwischen 20 °C und 110 °C und gegebenenfalls unter kontinuierlicher Entfernung des entstehenden Reaktionswassers oder des Reaktionsalkohols umgesetzt werden.

12. Verwendung der regioselektiven Hydroxyfettsäuren/-ester gemäß Ansprüche 1 bis 11 als Geliermittel, Emulgatoren, Dispergatoren, Stabilisatoren, Verdickungsmittel in technischen und kosmetischen Formulierungen.
